Europäisches Patentamt

European Patent Office

Office européen des brevets

(11) Publication number: **0 423 893 A1**

# EUROPEAN PATENT APPLICATION

(21) Application number: **90202743.2**

(22) Date of filing: **15.10.90**

(51) Int. Cl.⁵: **G06F 15/42**

(30) Priority: **16.10.89 NL 8902554**

(43) Date of publication of application:
**24.04.91 Bulletin 91/17**

(84) Designated Contracting States:
**DE FR GB NL**

(71) Applicant: **N.V. Nederlandsche**
**Apparatenfabriek NEDAP**
**Oude Winterswijkseweg 7**
**NL-7141 DE Groenlo(NL)**

(72) Inventor: **Hogen Esch, Johannes Harm Lukas**
**Hoge Veld 75**
**7122 ZN Aalten(NL)**

(74) Representative: **Smulders, Theodorus A.H.J.,**
**Ir. et al**
**Vereenigde Octrooibureaux Nieuwe Parklaan**
**107**
**NL-2587 BP 's-Gravenhage(NL)**

(54) **Information storage and monitoring system for health centres.**

(57) A method of storing and monitoring patient-related information in a health centre, wherein each
patient is provided with a patient-connected electronic data carrier which can be read out and re-
programmed, that the patient-related data are stored
both locally in the patient-connected electronic data
carrier and centrally in a central computer, and that
the locally stored data are compared in predeter-
mined situations with the centrally stored data and
that the two types of data are matched, if necessary.

FIG.1

## INFORMATION STORAGE AND MONITORING SYSTEM FOR HEALTH CENTRES.

This invention relates to a method of storing and monitoring patient-related information in a health centre, and to a system suitable for the use of this method.

At present, in the medical world medical data of a patient are recorded and monitored in different ways. An important phenomenon in hospitals is that the medical data of a certain patient are recorded in different locations in the hospital and on different media. A known option is the patient file attached to the foot of the bed. The link between the patient and this file at the foot of the bed is made by comparing the patient number of the file with the patient number, which, optionally encoded, is recorded on a bracelet worn by the patient. A large part of the total of medical information related to a patient, such as information on tests that have been run and about medication is stored in a central processing unit in the hospital.

Accordingly, for diagnosing, various kinds of data must be used of which a part is stored locally. It will be clear that such a procedure is laborious and increases the risk of errors or mix-ups. It can also be observed that the procedure described makes it more difficult to check, monitor and treat the patient, which is to be done on the basis of all medical data and cannot be carried out optimally because each time it takes a relatively long time to gather the relevant information.

Finally, in many cases only a part of the medical data can be kept up-to-date due to limited medical and nursing staff. The missing part of the information might for instance relate to whether a certain procedure, treatment or medication has been performed or not.

It is an object of the present invention to overcome the above-mentioned drawbacks and generally to provide an effective method of storing and monitoring information in health centres. To that effect, a method of the type described hereinabove is characterized according to the invention in that each patient is provided with a patient-connected electronic data carrier which can be read out and re-programmed, that the data related to each patient are stored both locally in the patient-connected electronic data carrier and centrally in a central computer, and that in predetermined situations the locally stored data are compared with the centrally stored data and that the two types of data are matched, if necessary.

A system for the application of the method is characterized according to the invention by a central computer where the patient-related data are centrally stored and at least one electronic data carrier that can be read out and re-programmed, where patient-related information is stored locally.

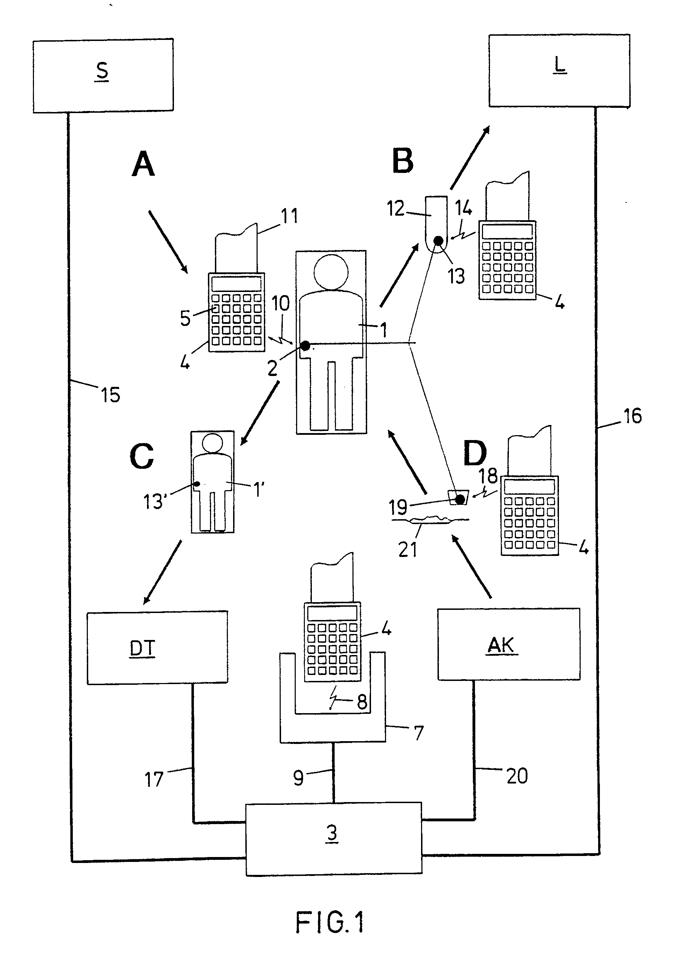

The invention will now be further explained and illustrated with reference to the accompanying drawing.

The single Figure schematically shows the data streams as they occur in respect of a patient in a health centre. The data storage and monitoring according to the invention is based on the possibility of local storage of data in a patient 1 in a programmable electronic data carrier, sometimes referred to as responder, for instance a bracelet that is hard to remove and comprises one or more integrated electronic circuits. Preferably, use is made here of contactless data transmission based on electromagnetic identification principles, as set forth by applicant in for instance NL Patent No. 176404 and NL patent application 8601021. In summary, the principle is that a responder comprising a coded electronic circuit provided with a resonance circuit is brought within an interrogation field generated by a transmitter-receiver. If the resonance circuit is attuned to the frequency of the interrogation field generated by the transmitter/receiver, the (coded) disturbance of the electromagnetic field caused by the responder can be recognized by the transmitter/receiver. Further, NL patent application 8601021 describes the principle which enables re- programming, i.e. modification of the code of the responder by means of the same transmitter/receiver. The code represents the data associated with the patient.

In the data storage and monitoring system according to the invention the patient is provided with a re-programmable electronic responder 2, for instance in the form of a bracelet. Recorded in this responder is first of all a unique identification number associated with the patient, optionally together with the personal details of the patient in question. The identification number and the personal details correspond to those stored in the central computer 3 of the hospital.

A number of procedures revolving around the patient will now be described successively. Of importance is that the data are stored locally, because procedures that are interrelated are not always carried out at the same time.

For the data transfer from a patient 1 to the central computer 3 and vice versa, preferably a portable hand terminal 4 comprising a keyboard 5 and a display 6 is used. The hand terminal can temporarily store information and transfer it, for instance in a combined load and data transfer apparatus 7, to the central computer 3, as indicated schematically by an arrow 8 and a line 9. The hand terminal 4 can also transfer data from and to the patient's data carrier formed by the responder, as schematically indicated by the arrow 10 and preferably offers the additional option of printing certain information on paper to permit visual checking in the case of malfunction of the apparatus. Preferably, all data transfer is contactless.

In the description of the procedures it is assumed that a single terminal is being used, but more terminals may be involved, for instance for the various departments.

Letter A schematically indicates a procedure where a specialist, schematically designated S, accompanied by nursing staff, comes to the patient to make a diagnosis and wishes to confirm this diagnosis on the basis of a number of blood tests to be run, for instance a glucose level assay. First of all, the data which relate to the identification of the patient and are stored in the data carrier are detected by means of the hand terminal and stored in the hand terminal. Then the desired laboratory tests and/or other tests to be run by means of diagnostic apparatus such as X-ray apparatus, ultrasound apparatus, CT (computer tomography) apparatus, are entered into the hand terminal by means of the keyboard 5 and stored.

Then all this information is written back into the idata carrier (responder) and, using the printer built into the hand terminal, a print 11 is made of this information, which is subsequently stored in the patient file.

After one or more patients have been visited in this manner, and the information associated with these patients has been stored in the hand terminal 4, this hand terminal is placed in the combined load and data transfer apparatus 7 and automatically all information is transferred to the central computer via a suitable line, schematically indicated at 9.

After this data transfer the information in the hand terminal is erased and thus the terminal is ready for use for other patients again.

The specialist in question can afterwards add any additional information to the patient data that are already stored in the central computer. This is schematically indicated by the line 15. The added or modified information must be reported to the department of the patient in question so that later verfication and matching is possible.

The hand terminal may be provided with for instance a sphygmomanometer, so that such information, too, can be transferred directly to the central computer without risk of confusion.

Procedure B concerns the gathering of body fluids, such as blood, necessary for laboratory tests. Beforehand, the identity and the medically relevant information of the patients to be visited, determined in procedure A, is read in from the central computer into the hand terminal. To that end, again use can be made of a load and data transfer apparatus 7. When the nursing staff comes to a certain patient, the operations to be performed, as determined in procedure A, are read in from the data carrier into the hand terminal and compared with the information coming from the central computer. In the case where additional information has been added or changes have been made, such information, after verification, is added or modified in the data carrier.

In the case where blood samples are taken, all relevant information is transferred to a data carrier 13 connected to the blood sample tube 12, as schematically indicated by an arrow 14. In this way the information is locally coupled to the blood sample in the tube 12 and the chance of a mix-up with blood samples of other patients is virtually nil. In the laboratory schematically indicated at L, the blood samples in question are often subjected to several assays. For that purpose the samples must often be divided. When such division is being effected, it is important that each time the relevant information is coupled locally to all divisional samples. The analyzing apparatus (not shown) is equipped with a reading device capable of reading out the information from the sample tubes 12, so that this analyzing apparatus can be controlled entirely by the local information in the data carriers 13 linked to the sample tubes. In this way, here, too, any mix-ups are precluded. In the analyzing apparatus, optionally linked to the central computer, as indicated with a line 16, verification can take place again, whereafter the measured values found are returned to the central computer again.

Further, these measuring values are printed by the analyzing apparatus or by the laboratory staff in a report. Procedure C concerns the diagnosis and/or treatment of the patient with, for instance, X-ray apparatus, CT apparatus, radiologic apparatus for irradiation or other apparatus. This is schematically represented by block DT. Here, coupled to this apparatus, the data carrier $13'$ can be read out into the apparatus of the transported patient $1'$ by means of a reading device and it can be verified whether the right patient is receiving the right treatment. Further, the results of the various diagnosis and treatment systems can be automatically linked to the identity of the patient. The relevant apparatus is preferably coupled to the central computer 3 via a line 17 and may be provided with a terminal. Optionally, a hand terminal can be used again and an intermediate station 7.

Procedure D concerns the line of distribution of medicines and food between the dispensary or kitchen AK and the patient. The medicines are for instance packed in appropriate containers 18 which are also provided with a data carrier 19. The relevant information is transferred from the central computer via a schematically indicated line 20 to the data carrier 19 of the container 18. At the patient end, by means of a hand terminal a verification takes place of the information in the medicine container and that of the patient. Via the hand terminal this verification can also be transferred to the central computer so that it is recorded whether this verification has taken place.

For the distribution of food 21, in the case of diets, the same procedure can be employed.

It is observed that after perusal of the above, various modifications will readily occur to anyone skilled in the art. Thus, for instance, there may be a fixed connection between the dispensary or kitchen, or diagnostic or therapeutic apparatus and the central computer, as shown, but the connection may also be effected through hand terminals and an apparatus such as the load and data transfer apparatus 7. Further the data carrier of a patient may be constructed as a bracelet with a special fastening which can only be undone with a special tool. Further, besides body fluids, also tissue specimens or x-rays and the like can be provided with local data carriers. These and similar modifications are considered to fall within the scope of the invention.

**Claims**

1. A method of storing and monitoring patient-related information in a health centre, characterized in that each patient is provided with a patient-connected electronic data carrier which can be read out and re-programmed, that the data related to each patient are stored both locally in the patient-connected electronic data carrier and centrally in a central computer, and that in predetermined situations the locally stored data are compared with the centrally stored data and that the two types of data are matched, if necessary.

2. A method according to claim 1, characterized in that for reading out from the the data carrier, temporarily storing, exchanging with the central computer and writing into a data carrier patient-related data, at least one hand terminal is used, which can communicate with the central computer via a load and data transfer device.

3. A method according to claim 1 or 2, characterized in that patient-related material intended for tests, is provided with at least one second electronic data carrier that can be read out and re-programmed, in which the patient-related data are stored.

4. A method according to claim 1 or 2, characterized in that medicines or foodstuffs intended for a patient are provided with at least one second electronic data carrier which can be read out and re-programmed.

5. A method according to claim 3 or 4, characterized in that for writing in and reading out and checking the data of said at least one second electronic data carrier, a hand terminal is used.

6. A method according to any one of the preceding claims, characterized in that diagnostic and/or therapeutic apparatus is provided with a terminal that is connected to the central computer for data exchange with the central computer and with a patient-connected data carrier.

7. A method according to any one of the preceding claims, characterized in that the doctor in attendance writes information about the treatments and/or tests to be performed into a patient's data carrier by means of a hand terminal.

8. A system for storing and monitoring patient-related information in a health centre, characterized by a central computer in which patient-related data are centrally stored and at least one electronic data carrier that can be read out and re-programmed, in which patient-related data are stored locally.

9. A system according to claim 8, characterized by at least one portable hand terminal which can exchange data with both said at least one data carrier and the central computer.

10. A system according to claim 9, characterized by at least one load and data transfer device via which said at least one hand terminal can communicate with the central computer.

11. A system according to any one of claims 8-10, characterized in that for each patient a data carrier is provided and that for patient-related test material at least one second data carrier is provided.

12. A system according to any one of claims 8-11, characterized in that for medicines or food intended for a patient a data carrier is provided.

13. A system according to any one of claims 9-12, characterized in that the data carrier(s) and said at least one hand terminal are equipped for contactless data exchange.

14. A system according to any one of claims 9-13, characterized in that said at least one hand terminal is provided with a printer for printing data on paper.

15. A system according to any one of claims 8-14, characterized by diagnostic and/or therapeutic apparatus which is connected with the central computer and provided with a reading device for reading the data stored in a data carrier.

16. A system according to any one of claims 8-15, characterized by analyzing apparatus for analyzing material coming from a patient, which analyzing apparatus is provided with a reading device for reading data stored in a data carrier.

17. A system according to any one of claims 8-16, characterized in that the dispensary and/or the kitchen of the health centre are connected with the central computer and are provided with a device for writing data in a data carrier that can be re-programmed and read out.

18. A system according to any one of claims 8-17, characterized in that one of the data carriers connected with a patient is constructed as a bracelet with a special fastening.

FIG.1

European
Patent Office

**EUROPEAN SEARCH
REPORT**

Application Number

**EP 90 20 2743**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 700 659  (CLINICOM) <br> * Page 2, line 30 - page 3, line 33; page 6, line 9 - page 7, line 34 * <br><br> — — — — — | 1-6,8-18 | G 06 F <br> 15/42 |

TECHNICAL FIELDS
SEARCHED (Int. Cl.5)

G 06 F 15/42

The present search report has been drawn up for all claims

| Place of search | Date of completion of search | Examiner |
|---|---|---|
| The Hague | 31 January 91 | BURGAUD C. |